# EUROPÄISCHE PATENTSCHRIFT

(11) **EP 1 092 970 B1**
(45) Veröffentlichungstag und Bekanntmachung des Hinweises auf die Patenterteilung: **22.09.2004**
(21) Anmeldenummer: 00121326.3
(22) Anmeldetag: 09.10.2000
(51) Int. Cl.: G01N 13/02, G01N 33/14

(54) **Verfahren und Vorrichtung zum Messen der Schaumeigenschaften von Flüssigkeiten**
Method and device for measuring the foaming properties of liquids
Dispositif de caractérisation des propriétés moussantes de liquides

(30) Priorität: 16.10.1999 DE 19949922
(43) Veröffentlichungstag der Anmeldung: 18.04.2001
(73) Patentinhaber: SITA Messtechnik GmbH, 01217 Dresden (DE)
(72) Erfinder: Perrin, Frank, Dipl.-Ing., 01900 Kleinröhrsdorf (DE)
(74) Vertreter: Ilberg, Roland W., Dipl.-Ing.

(56) Entgegenhaltungen:
- DE-A- 4 036 344
- SHI T M ET AL: "CAPACITANCE-BASED INSTRUMENTATION FOR MULTI-INTERFACE LEVEL MEASUREMENT" MEASUREMENT SCIENCE AND TECHNOLOGY, IOP PUBLISHING, BRISTOL, GB, Bd. 2, Nr. 10, 1. Oktober 1991 (1991-10-01), Seiten 923-933, XP000264319 ISSN: 0957-0233
- PERRIN, F; JUNG, U: "Schaum - nicht nur wichtig für gutes Bier" LABORPRAXIS, März 2000 (2000-03), Seiten 60-65, XP002259283

## Beschreibung

Die Erfindung bezieht sich auf ein Verfahren und eine Vorrichtung zum Messen der Schaumeigenschaften von Flüssigkeiten.

Der Messung des Schaumvermögens von Flüssigkeiten, kommt in der technischen und Lebensmittelindustrie große Bedeutung zu, um die Qualität der schäumbaren Flüssigkeiten, beispielsweise von Färb- und Reinigungslösungen, Galvanikbädern, Emulsionen, Spülmitteln, Körperpflegemitteln, aber auch z.B. im Bier usw. zu bestimmen.

Nach DIN-Norm 53 902, Teil 1 wird hierzu der Schaum durch 30 Sekunden langes Schlagen der flüssigen Probe in einem transparenten, skalierten Messzylinder erzeugt und anschließend die Schaumhöhe nach Augenmaß abgeschätzt.

Es ist an dieser Stelle festzuhalten, dass Schaum keineswegs eine ebene Oberfläche bildet, sondern insbesondere an den Rändern eines Gefäßes beträchtlich vom übrigen Niveau-Mittelwert abweichen kann und auch sonst über seine Oberfläche verteilt Schaumberge und Schaumsenken ausbildet. Das Ablesen ist damit weitgehend abhängig von subjektiven Faktoren und eignet sich in keiner Weise für eine Automatisierung.

Auch nach DE 197 40 095 A1 wird die Schaumhöhe abgelesen, wobei der Schaum durch Rühren mittels eines speziellen Rührers bei vorgegebener Drehzahl und Rührzeit erzeugt wird.

Nach der DE 40 36 344 C2 wird der Schaum einer Probe durch Umwälzen der Probe in einem doppelwandigen Gefäß über eine externe Umwälzpumpe erzeugt, indem beim Aufprallen eines dünnen Flüssigkeitsstrahls auf eine Prallplatte die Flüssigkeit intensiv verwirbelt und verschäumt wird. Mit Hilfe einer auf dem Schaum gelagerten Höhensensorplatte und deren Positionsfeststellung kann die Schaumhöhe gemessen werden, wobei das Gewicht der Höhensensorplatte mittels eines elektrischen Antriedsmotors geregelt kompensiert wird, damit nur ein der Festigkeit des Schaums angepasstes Restgewicht der Höhensensorplatte wirksam bleibt. Zur kontinuierlichen Messung der Höhensensorplattenposition dient ein inkrementaler Winkelgeber auf der Achse des elektrischen Antriebs, der die Winkelposition der das Tragseil für die Höhensensorplatte aufwickelnden Seilführungsscheibe auf der Antriebsachse bestimmt. Ein angeschlossener Rechner kann die Schaumzerfallphase automatisch aufzeichnen. Neben dem hohen Messaufwand ist als entscheidender Nachteil dieser Messmethode zu nennen, dass die Höhensensorplatte einen nicht unerheblichen Einfluss auf die Schaumoberfläche ausübt, indem mehr oder weniger Schaumbläschen zerstört werden, wodurch der Schaum weitaus schneller zerfällt als ohne mechanische Beeinträchtigung durch die Höhensensorplatte. Ist die Höhensensorplatte etwa querschnittsgleich mit der Schaumoberfläche, verfälschen wiederum die Randzonen das Messergebnis, ist die Höhensensorplatte hingegen dementsprechend kleiner als die Schaumoberfläche, so gibt es Zonen, die von der Höhensensorplatte beeinträchtigt werden und Zonen, bei denen sich der Schaum unbeeinträchtigt halten kann.

Nach DE 37 12 377 C2 wird der Schaum mit vorgegebener Drehzahl eines Rührers in einem Messzylinder erzeugt, der in einem Becher für die Messflüssigkeit hängt. Als Messeinrichtung dient eine Elektrode, die im Messzylinder oberhalb des Flüssigkeitsspiegels in der Schaumdecke angeordnet ist und mit einer Gegenelektrode am Grunde des Bechers zusammenarbeitet. Legt man an die beiden Elektroden eine elektrische Spannung an, so fließt nur dann ein Strom, wenn oberhalb des Flüssigkeitsspiegels die Schaumdecke den Stromkreis schließt. Bricht die Schaumdecke nach ihrer Erzeugung soweit zusammen, dass kein Kontakt mehr zwischen der Schaumdecke und der oberen Elektrode besteht, so wird der Stromkreis unterbrochen. Die Zeit, in der zwischen den beiden Elektroden ein Strom fließt, kann als Maß tür die Tensidkonzentration dienen.

Diese einfache Messvorrichtung setzt voraus, dass die Messflüssigkeit eine gewisse elektrische Leitfähigkeit besitzt, was in den meisten praktisch vorkommenden Fällen zutrifft. Allerdings ist es mit vorgenannter Messeinrichtung nicht möglich, das maximal ausgebildete Schaumvolumen zu messen, da die Schaumentwicklung bis über die obere Elektrode erfolgt.

Aus der DE 42 08 598 A1 ist es auch schon bekannt, zwei Elektroden in gleicher Höhe in einem Behälter fest zu installieren, wobei über beide Elektroden bei Schaumbildung oder steigendem Flüssigkeitspegel getrennte Stromkreise mit unterschiedlich großen Bürden in den Messkreisen geschlossen werden. Hierdurch kann detektiert werden, ob nur der Schaum oder auch die Flüssigkeit einen vorgegebenen Pegel überschritten haben, da der ohmsche Widerstand über die Schaumschicht ungleich größer als durch die tensidhaltige Flüssigkeit ist.
Der Erfindung liegt die Aufgabe zugrunde, ein Verfahren und eine Vorrichtung zum Messen der Schaumeigenschaften einer Flüssigkeit zu schaffen, wobei neben einer Automatisierung insbesondere das exakte Messen des Schaum-Oberflächenprofils und/oder der Schaumhöhe einer aufgeschäumten Flüssigkeit im Vordergrund steht.

Die Aufgabe wird durch ein Verfahren nach Anspruch 1 oder 2 gelöst. Eine Vorrichtung zum Messen gibt Anspruch 4 an. Anwendungen und vorteilhafte Weiterbildungen der Erfindung geben die begleitenden Unteransprüche 3 und 5- 13 an.

Durch die Erfindung werden sämtliche subjektive Messfehler ausgeschlossen, da die Erfassung des Schaumoberflächenprofils bzw. der Schaumhöhe vollautomatisch und mit praktisch beliebig wählbarer Genauigkeit erfolgt. Zugleich wird mit der Automatisierung ein wesentlicher Einsparungseffekt erzielt, der insbesondere bei kontinuierlich zu messenden Prozessverhältnissen sich positiv auf die Wirtschaftlichkeit auswirkt.

Die Erfindung soll anhand eines Ausführungsbeispiels näher erläutert werden. In der zugehörigen Zeichnung zeigt:
Fig. 1 eine Prinzipdarstellung einer Messvorrichtung im Schnitt und
Fig. 2 eine Prinzipdarstellung einer Messelektrodenanordnung in der Draufsicht.

Es sei vorangestellt, dass die Eigenschaften eines Schaums wesentlich von der Art und Weise der Schaumerzeugung abhängen. Wird der Schaum beispielsweise durch Rühren der Flüssigkeit in einem Gefäß erzeugt, so hängt die Menge und die Konsistenz des gebildeten Schaums außer von z.B. der Tensidkonzentration auch von der Drehzahl eines Rührers, der oder den Rührzeiten, der Geometrie des Rührers, der Geometrie des Messgefäßes, der Temperatur der Flüssigkeit u.a. ab. Sofern diese äußeren Randbedingeungen zur Reproduzierbarkeit der Bestimmung der Schaumeigenschaften unverändert beibehalten werden, hängen die Eigenschaften des gebildeten Schaums nur noch vom Schaumbildungsvermögen ab und es ist unerheblich, auf welche Weise der Schaum letztlich konditioniert wird.

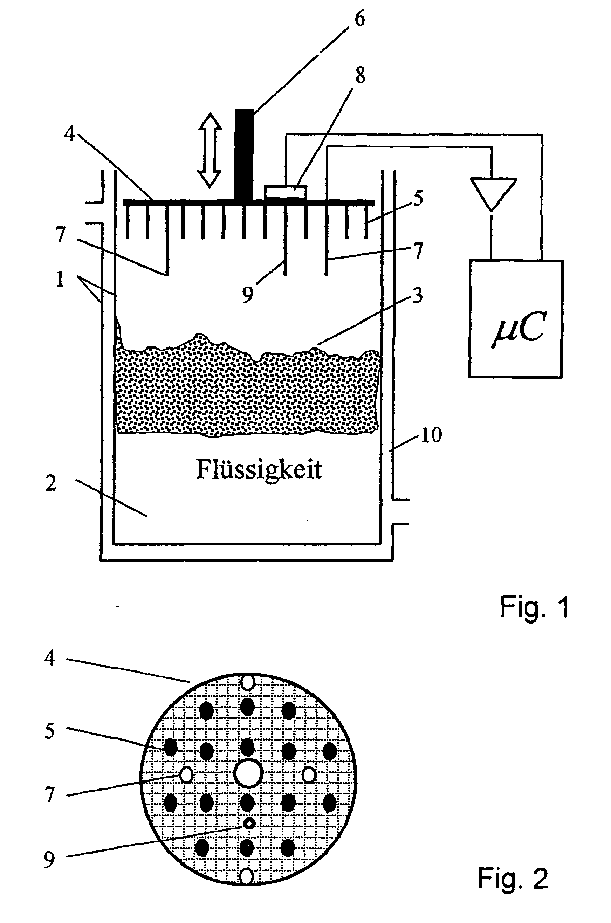

Fig. 1 zeigt im Schnitt einen doppelwandigen Messzylinder 1 einer Vorrichtung mit einer vorgegebenen Menge von Messflüssigkeit 2, die nach einem beliebigen, reproduzierbaren Verfahren aufgeschäumt ist. Einzelheiten einer Verschäumungsvorrichtung können deshalb zwecks besserer Übersichtlichkeit weggelassen werden.

Über der in aller Regel unebenen Schaumoberfläche 3 befindet sich eine Tragplatte 4 der Vorrichtung mit einer Vielzahl gleichlanger elektrisch gegeneinander isolierter Elektroden 5 als Sensoren. Die Tragplattte 4 ist mit einer nicht näher dargestellten automatischen Absenkeinrichtung 6 der Vorrichtung verbunden. Beim Absenken der Tragplatte 4 werden die Elektroden 5 mit dem Schaum kontaktieren und zwar je nach der Höhe des Schaums genau unterhalb jeder Elektrode 5. So wird eine Elektrode 5 zuerst, das heißt mit weitestem Abstand der Tragplatte 5 zur Flüssigkeitsoberfläche bzw. einem beliebig anderen Bezugspunkt, mit der Schaumoberfläche 3 in Berührung kommen, dann nacheinander weitere und letztlich alle. Dementsprechend werden nacheinander separate Stromkreise über jede einzelne Elektrode 5 und eine Auswerteeinrichtung (Mikrocomputer) zu mindestens einer Gegenelektrode 7 geschlossen, die beispielsweise ebenfalls auf der Tragplatte 4 angeordnet ist und aufgrund ihrer Erstreckung zuerst mit dem Schaum kontaktiert. Die Zeitverzögerung oder der unterschiedliche Weg der Elektroden 5 bis zur Erstberührung der Schaumoberfläche 3 werden in dem Mikrorechner µC erfasst, ausgewertet und ausgegeben bzw. abrufbar gespeichert. Im Ergebnis ist damit das genaue Oberflächenprofil und damit die Oberflächenbeschaffenheit der Schaumoberfläche 3 und hieraus bei Bedarf ein statistischer Mittelwert für die Schaumhöhe ablesbar, bzw. als Stell- oder Regelgröße für automatische Zudosierungsprozesse von Tensiden o. ä. Zusätzen weiterverarbeitbar.

Um den Aufbau der Vorrichtung und die Auswertung der Signale von den Elektroden 5 zu vereinfachen, werden alle Anschlüsse der Elektroden 5 auf einen z.B. 300 Hz-Multiplexer 8 mit 16 Abgängen gegeben, der zweckmäßigerweise ebenfalls auf der Tragplatte sitzt und die Elektroden 5 nacheinander im Takt nach Kontaktierungen mit der Schaumoberfläche 3 abfragt. Die Signale werden verstärkt dem Eingang des Mikrorechners µC zugeführt, der auch den Multiplexer steuert. Es versteht sich, dass bei größeren Schaumoberflächen mehr als ein Multiplexer eingesetzt werden kann.

Außerdem sitzt auf der Tragplatte 4 ein Temperaturfühler 9, der für eine reproduzierbare Temperatur des Mess-Schaurns im Zusammenhang mit einer nicht näher beschriebenen Temperaturregelungsschaltung sorgt, die Einfluss auf ein Temperierungsmedium 10 nimmt, das über den Ein- und Auslass des doppelwandigen Messzylinders 1 zirkuliert.

In Fig. 2 ist eine Tragplatte 4 der Vorrichtung mit Elektroden 5, Gegenelektroden 7 und einem Temperaturfühler 9 stark schematisiert in der Draufsicht dargestellt. Die Elektroden 5 sind in einem Raster angeordnet, hier in karthesischen Koordinaten. Ebenso könnte das Raster für insbesondere zylindrische Messgefäße Polarkoordinaten folgen oder nach Art von Bienenwaben ausgeführt sein. Das Raster hat die Funktion, die Koordinaten einer jeden Elektrode 5 besonders einfach zu verarbeiten. Es versteht sich, dass ein Mikroprozessor auch jede andere Verteilung der Elektroden 5 auf der Elektrodenplatte 4 verarbeiten kann. Praktisch haben sich wenige Gegenelektroden 7 als ausreichend erwiesen, jedoch kann auch jeder Elektrode 5 eine Gegenelektrode 7 zugewiesen sein.

Die Anzahl der Elektroden 5 ist sehr klein gegenüber der Anzahl der Schaumbläschen auf der Schaumoberfläche. Somit verfälschen die bei einer Kontaktierung zerstörten Bläschen nicht signifikant das Messergebnis. Andererseits sind so viele Elektroden 5 auf der Tragplatte 4 angeordnet, dass sich ein sehr gutes Bild über die Oberflächenstruktur des Schaums erstellen lässt. Die Flachenauflösung (Anzahl der Elektroden/ Flächeneinheit) ist u.a. von der Art des Schaums und seiner Erzeugung abhängig. In der Praxis erweisen sich kleiner 1 bis 5 Elektroden pro cm² Messoberfläche als durchaus befriedigend.

Die Größe der Absenkschritte der Trägerplatte 4 bestimmt gemeinsam mit der Flächenauflösung die räumliche Auflösung der Messung. Im Allgemeinen ist eine Höhenabsenkung in 0,5 mm- bis 2mm- Schritten ausreichend, um eine Schaumoberfläche abzutasten.

Die Tragplatte 4 kann die gesamte Schaumoberfläche 3 überdecken, also auch die kritischen Wandungsbereiche, in denen der Schaum aufgrund von Grenzflächeneffekten beträchtlich höher oder unter Umständen auch tiefer steht als weiter im Inneren des Messzylinders 1, da der Wandungsbereich rechnerisch berücksichtigt werden kann, sie kann aber auch einen etwas kleineren Flächeninhalt haben.

Nach einer weiteren Ausführung kann die Tragplatte mit den Sensoren 5 zunächst soweit abgesenkt werden, dass alle Sensoren 5 mit dem Schaum kontaktieren und danach jeder Kontaktverlust eines Sensors 5 mit der Schaumoberfläche über einen definierten Aufwärtshub der Tragplatte 4 für sich erfasst und ausgewertet werden.

Das vorgestellte Verfahren und die vorgenannte Einrichtung eignen sich beispielsweise für die Ermittlung statischer Schaumoberflächen, dass heißt, es wird angenommen, dass sich die Schaumoberfläche innerhalb der Messzeit nicht wesentlich verändert. Dies ist bei vielen Schäumen der Fall.

Das Verfahren kann ebenso für eine dynamische Oberflächenmessung von Schäumen herangezogen werden, indem mehrere Messungen mit jeweils aufs Neue abgesenkter Tragplatte 4 in bestimmten Zeitintervallen erfolgen, oder die Tragplatte 4 wird soweit abgesenkt, bis alle Sensoren 5 mit dem Schaum kontaktieren, danach festgestellt und der Zeitpunkt erfasst, an dem die Kontakte abreißen. Daraus lässt sich jeweils der Zerfallsprozess des Schaums ableiten.

Weiterhin eignen sich das Verfahren und die Vorrichtung für Maximum-Minimum-Messungen der Schaumoberfläche.

## Patentansprüche

1. Verfahren zum Messen der Schaumeigenschaften von Flüssigkeiten mit Hilfe einer Sensoranordnung,
**dadurch gekennzeichnet,**
**dass** eine Vielzahl flächig über einer Schaumoberfläche (3) verteilter Sensoren (5) auf die Schaumoberfläche (3) definiert abgesenkt wird und jede Kontaktierung eines Sensors (5) mit der Schaumoberfläche (3) für sich erfasst und ausgewertet wird.

2. Verfahren zum Messen der Schaumeigenschaften von Flüssigkeiten mit Hilfe einer Sensoranordnung,
**dadurch gekennzeichnet,**
**dass** eine Vielzahl flächig über einer Schaumoberfläche (3) verteilter Sensoren (5) in den Schaum eingesetzt wird und jeder Kontaktverlust eines Sensors (5) mit der Schaumoberfläche (3) über die Zeit und/oder einen definierten Aufwärtshub der Sensoren (5) für sich erfasst und ausgewertet wird.

3. Verfahren nach Anspruch 1 oder 2,
**dadurch gekennzeichnet,**
**dass** das Absenken bzw. das Heben der Sensoren (5) schrittweise erfolgt.

4. Vorrichtung zum Messen der Schaumeigenschaften von Flüssigkeiten mit einer Sensoranordnung und einem Messgefäß zur Aufnahme der Flüssigkeit, insbesondere für ein Verfahren nach Anspruch 1 oder 2,
**dadurch gekennzeichnet,**
**dass** eine Tragplatte (4) der Vorrichtung mit einer Vielzahl von flächig über einer zu messenden Schaumoberfläche (3) der aufgeschäumten Flüssigkeit verteilten, nadelförmigen, vertikal ausgerichteten und gegeneinander elektrisch isolierten Elektroden (5) versehen ist und mittels einer automatischen Senk-/Hebe-Einrichtung (6) der Vorrichtung in bezug zur Schaumoberfläche (3) der in dem Messgefäß (1) befindlichen Flüssigkeit (2) absenkbar, hebbar und/oder feststellbar ist, wobei jede einzelne Kontaktierung/ Dekontaktierung eines Sensors (5) mit der Schaumoberfläche (3) mit der Vorrichtung automatisch erfassbar und zur Bestimmung des Schaumoberflächenprofils und/oder der Schaumhöhe auswertbar ist.

5. Vorrichtung nach Anspruch 4,
**dadurch gekennzeichnet,**
**dass** die Elektroden (5) in einem Raster angeordnet sind.

6. Vorrichtung nach Anspruch 4 oder 5,
**dadurch gekennzeichnet,**
**dass** die Anzahl der Elektroden (5) auf der Tragplatte (4) zwischen kleiner 1 und 5 je cm² Messoberfläche beträgt.

7. Vorrichtung nach mindestens einem der Ansprüche 4 bis 6,
**dadurch gekennzeichnet,**
**dass** die Elektroden (5) gleichlang sind.

8. Vorrichtung nach Anspruch 4 oder 7,
**dadurch gekennzeichnet,**
**dass** auf der Tragplatte (4) mindestens eine Gegenelektrode (7) angeordnet ist und die Gegenelektrode(n) (7) länger als die Elektroden (5) ist/ sind.

9. Vorrichtung nach Anspruch 4,
**dadurch gekennzeichnet,**
**dass** das Messgefäß (1) der Vorrichtung temperiert ist.

10. Vorrichtung nach Anspruch 9,
**dadurch gekennzeichnet, dass** auf der Tragplatte (4) ein Temperaturfühler (9) angeordnet ist.

11. Vorrichtung nach mindestens einem der vorstehenden Ansprüche 4 bis 10,
**dadurch gekennzeichnet,**
**dass** auf der Tragplatte (4) ein Multiplexer (8) sitzt, der den Zustand der Elektroden (5) zeitlich zueinander versetzt an einen Mikrorechner sendet.

12. Verwendung einer Vorrichtung nach mindestens einem der Ansprüche 4 bis 11 zur dynamischen Messung der Schaumeigenschaften einer Flüssigkeit.

13. Verwendung einer Vorrichtung nach mindestens einem der Ansprüche 4 bis 11 zur statischen Messung der Schaumeigenschaften einer Flüssigkeit.

## Claims

1. Method for measuring the foam properties of liquids with the aid of a sensor arrangement, **characterised in that** a large number of sensors (5) distributed in a planar manner over a foam surface (3) are lowered in a defined manner onto the foam surface (3) and each contact of a sensor (5) with the foam surface (3) is detected and evaluated separately.

2. Method for measuring the foam properties of liquids with the aid of a sensor arrangement, **characterised in that** a large number of sensors (5) distributed in a planar manner over a foam surface (3) are inserted into the foam and each loss of contact of a sensor (5) with the foam surface (3) over the time and/or a defined upward lift of the sensors (5) is detected and evaluated separately.

3. Method according to claim 1 or 2, **characterised in that** the sensors (5) are lowered or lifted stepwise.

4. Device for measuring the foam properties of liquids with a sensor arrangement and a measuring vessel for receiving the liquid, in particular for a method according to claim 1 or 2, **characterised in that** a support plate (4) of the device is provided with a large number of needle-shaped, vertically oriented electrodes (5) electrically insulated from one another and distributed in a planar manner over a foam surface (3) of the foamed liquid to be measured and can be lowered, lifted and/or established by means of an automatic lowering/lifting apparatus (6) of the device with respect to the foam surface (3) of the liquid (2) located in the measuring vessel (1), wherein each individual contact/loss of contact of a sensor (5) with the foam surface (3) can be automatically detected by the device and evaluated to determine the foam surface profile and/or the foam level.

5. Device according to claim 4, **characterised in that** the electrodes (5) are arranged in a grid.

6. Device according to claim 4 or 5, **characterised in that** the number of electrodes (5) on the support plate (4) is between less than 1 and 5 per cm² measuring surface.

7. Device according to at least one of claims 4 to 6, **characterised in that** the electrodes (5) are of equal length.

8. Device according to claim 4 or 5, **characterised in that** at least one counter-electrode (7) is arranged on the support plate (4) and the counter-electrode(s) (7) is/are longer than the electrodes (5).

9. Device according to claim 4, **characterised in that** the measuring vessel (1) of the device is temperature-controlled.

10. Device according to claim 9, **characterised in that** a temperature sensor (9) is arranged on the support plate (4).

11. Device according to at least one of the preceding claims 4 to 10, **characterised in that** a multiplexer (8) is located on the support plate (4) and transmits the state of the electrodes (5) in a temporally offset manner with respect to one another to a microcomputer.

12. Use of a device according to at least one of claims 4 to 11 for the dynamic measurement of the foam properties of a liquid.

13. Use of a device according to at least one of claims 4 to 11 for the static measurement of the foam properties of a liquid.

## Revendications

1. Procédé de mesure des propriétés moussantes de liquides à l'aide d'un ensemble de capteurs,
**caractérisé en ce qu'**
une pluralité de capteurs (5) répartis de manière plane au-dessus d'une surface de mousse (3) est descendue de manière définie sur la surface de mousse (3) et chaque contact d'un capteur (5) avec la surface de mousse (3) est relevé et exploité individuellement.

2. Procédé de mesure des propriétés moussantes de liquides à l'aide d'un ensemble de capteurs,
**caractérisé en ce qu'**
une pluralité de capteurs (5) répartis de manière plane au-dessus d'une surface de mousse (3) est introduite dans la mousse et chaque perte de contact d'un capteur (5) avec la surface de mousse (3) est relevée et exploitée individuellement pendant une durée et/ou une course montante définie des capteurs (5).

3. Procédé selon la revendication 1 ou 2,
**caractérisé en ce que**
la descente ou la montée des capteurs (5) se fait par paliers.

4. Dispositif de mesure des propriétés moussantes de liquides avec un ensemble de capteurs et un récipient de mesure pour recevoir le liquide, notamment pour un procédé selon la revendication 1 ou 2,
**caractérisé en ce qu'**
une plaque de support (4) du dispositif est munie d'une pluralité d'électrodes (5) réparties de manière plane au-dessus d'une surface de mousse (3) à mesurer du liquide moussé, en forme d'aiguilles, orientées verticalement et électriquement isolées les unes par rapport aux autres, et pouvant être descendue, montée et/ou immobilisée au moyen d'une installation de descente/ montée (6) du dispositif par rapport à la surface de mousse (3) du liquide (2) présent dans le récipient de mesure (1), chaque mise en contact/perte de contact d'un capteur (5) avec la surface de mousse (3) pouvant être relevée automatiquement par le dispositif et exploitée pour déterminer le profil de la surface de mousse et/ou la hauteur de la mousse.

5. Dispositif selon la revendication 4,
**caractérisé en ce que**
les électrodes (5) dans disposées selon une trame.

6. Dispositif selon la revendication 4 ou 5,
**caractérisé en ce que**
le nombre d'électrodes (5) sur la plaque de support (4) est compris entre inférieur à 1 et 5 par cm² de surface de mesure.

7. Dispositif selon au moins l'une quelconque des revendications 4 à 6,
**caractérisé en ce que**
les électrodes (5) présentent la même longueur.

8. Dispositif selon la revendication 4 ou 7,
**caractérisé en ce qu'**
au moins une contre-électrode (7) est disposée sur la plaque de support (4) et la (les) contre-électrode(s) est (sont) plus longue(s) que les électrodes (5).

9. Dispositif selon la revendication 4,
**caractérisé en ce que**
le récipient de mesure (1) du dispositif est tempéré.

10. Dispositif selon la revendication 9,
**caractérisé en ce qu'**
une sonde de température (9) est disposée sur la plaque de support (4).

11. Dispositif selon au moins l'une quelconque des revendications précédentes,
**caractérisé en ce que**
sur la plaque de support (4) se situe un multiplexeur (8) qui envoie l'état des électrodes (5) de manière décalée dans le temps vers un microcalculateur.

12. Utilisation d'un dispositif selon au moins l'une quelconque des revendications 4 à 11, pour la mesure dynamique des propriétés moussantes d'un liquide.

13. Utilisation d'un dispositif selon au moins l'une quelconque des revendications 4 à 11, pour la mesure statique des propriétés moussantes d'un liquide.
